# FASCICULE DE BREVET EUROPEEN

(11) **EP 1 087 961 B1**
(45) Date de publication et mention de la délivrance du brevet: **14.08.2002**
(21) Numéro de dépôt: 99923710.0
(22) Date de dépôt: 10.06.1999
(51) Int. Cl.: C07D 401/04, C07D 401/06, C07D 307/42, C07D 405/14, C07D 303/22, A61K 31/44

(54) **PHENOXYPROPANOLAMINES, PROCEDE POUR LEUR PREPARATION ET COMPOSITIONS PHARMACEUTIQUES LES CONTENANT**
PHENOXYPROPANOLAMIN-DERIVATE, VERFAHREN ZU IHRER HERSTELLUNG UND DIESE ENTHALTENDEN PHARMAZEUTISCHEN ZUSAMMENSETZUNGEN
PHENOXYLPROPANOLAMINES, METHOD FOR THE PRODUCTION THEREOF AND PHARMACEUTICAL COMPOSITIONS CONTAINING THE SAME

(30) Priorité: 18.06.1998 FR 9807660
(43) Date de publication de la demande: 04.04.2001
(73) Titulaire: SANOFI-SYNTHELABO, 75013 Paris (FR)
(72) Inventeur: CECCHI, Roberto, I-20075 Lodi (IT); CROCI, Tiziano, I-20155 Milano (IT); GUZZI, Umberto, I-20148 Milano (IT); MARSAULT, Eric, Sherbrooke, Quebec JIJ IJ4 (CA)
(74) Mandataire: Varady, Peter
(86) Numéro de dépôt international: FR9901370
(87) Numéro de publication internationale: WO9965895

(56) Documents cités:
- EP-A- 0 052 072
- WO-A-95/07274
- FR-A- 2 567 885
- M. HORI ET AL: "A soluble polymer approach to the "fishing out" principle: synthesis and purification of beta-amino alcohols" JOURNAL OF ORGANIC CHEMISTRY., vol. 63, no. 3, 6 février 1998 (1998-02-06), pages 889-894, XP002110346 AMERICAN CHEMICAL SOCIETY. EASTON., US ISSN: 0022-3263 cité dans la demande

## Description

La présente invention concerne de nouvelles phénoxypropanolamines, les compositions pharmaceutiques les contenant, un procédé pour leur préparation et des intermédiaires dans ce procédé.

BE 902897 décrit des aryloxypropanolamines portant un groupe 4-pipéridininyl-1-substitué sur l'amine, ces composés ayant une activité β₁-bloquante et α-bloquante. J. Org. Chem., 1998, 63:889:894, décrit d'autres aryloxypropanolamines portant un groupe 4- pipéridinyl-1-substitué sur l'aminé.

Il a été maintenant trouvé que des phénoxypropanolamines portant un radical 1-(pyrid-2-yl)-pipéridin-4-yl sur l'amine possèdent une activité agoniste vis-à-vis des récepteurs β₃-adrénergiques.

Ainsi, la présente invention concerne, selon un de ses aspects, des phénoxypropanolamines de formule (Ia) 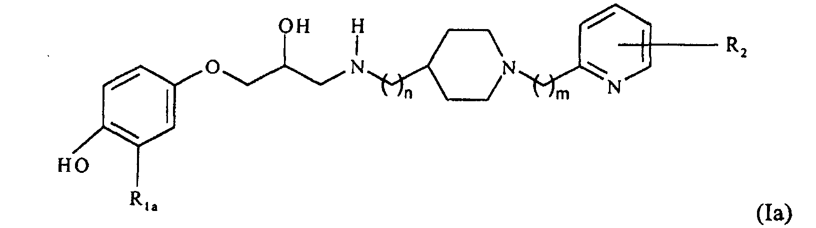
R₁ₐ représente l'hydrogène, un groupe -S(O)_{z}-(C₁-C₄)Alk, un groupe -CO(C₁-C₄)Alk, un groupe -NHSO₂-(C₁-C₄)Alk, un groupe NHCO(C₁-C₄)Alk, un groupe 2-furyle ou un halogène;
R₂ représente l'hydrogène ou un groupe (C₁-C₄)Alk, un groupe (C₁-C₄)alcoxyle, un halogène, -COOH, -COO(C₁-C₄)Alk, -CN, -CONR₃R₄, -NO₂, -SO₂NH₂, -NHSO₂(C₁-C₄)Alk;
   m et n indépendamment 0, 1 ou 2;
R₃ et R₄ représentent indépendamment l'hydrogène ou un groupe (C₁-C₄)Alk;
   Z est 1 ou 2;
et leurs sels ou solvates.

Selon un autre de ses aspects, l'invention concerne des composés de formule (I): 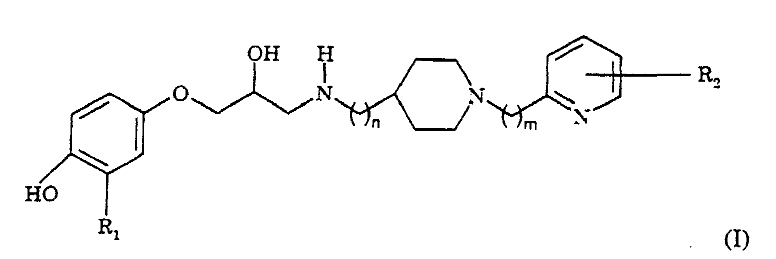 où
R₁ représente l'hydrogène, un groupe -S(O)_{z}-(C₁-C₄)Alk, un groupe -CO(C₁-C₄)Alk ou un groupe -NHSO₂-(C₁-C₄)Alk;
R₂ représente l'hydrogène ou un groupe (C₁-C₄)Alk, un groupe (C₁-C₄)alcoxyle, un halogène, -COOH, -COO(C₁-C₄)Alk, -CN, -CONR₃R₄, -NO₂, -SO₂NH₂, -NHSO₂(C₁-C₄)Alk;
   m et n indépendamment 0, 1 ou 2;
R₃ et R₄ représentent indépendamment l'hydrogène ou un groupe (C₁-C₄)Alk;
Z est 1 ou 2;
et leurs sels ou solvates.

Dans la présente description le terme "(C₁-C₄)Alk" désigne un radical monovalent d'un hydrocarbure en C₁-C₄ saturé à chaîne droite ou ramifiée.

Les sels des composés de formule (I) et (Ia) selon la présente invention comprennent aussi bien les sels d'addition avec des acides minéraux ou organiques pharmaceutiquement acceptables tels que le chlorhydrate, le bromohydrate, le sulfate, l'hydrogénosulfate, le dihydrogénophosphate, le citrate, le maléate, le tartrate, le fumarate, le gluconate, le méthanesulfonate, le 2-naphtalènesulfonate, etc., que les sels d'addition qui permettent une séparation ou une cristallisation convenable des composés de formule (I) ou (Ia) tels que le picrate, l'oxalate ou les sels d'addition avec des acides optiquement actifs, par exemple les acides camphorsulfoniques et les acides mandéliques ou mandéliques substitués.

De plus, lorsque les composés de formule (I) et (Ia) possèdent un groupe carboxy libre les sels comprennent aussi les sels avec des bases minérales, de préférence celles avec des métaux alcalins tels que le sodium ou le potassium, ou avec des bases organiques.

Les stéréoisomères optiquement purs, ainsi que les mélanges d'isomères des composés de formule (I) et (Ia), dus aux carbones asymétriques ou au groupe sulfinyle dans la signification de R₁ₐ ou R₁, dans une proportion quelconque, sont partie de la présente invention.

Des composés préférés de la présente invention comprennent les composés de formule (I) ou (Ia) où le groupe R₂ est dans la position 5 de la pyridine.

D'autres composés préférés comprennent les composés de formule (I) ou (Ia) où le groupe R₂ est dans la position 6 de la pyridine.

D'autres composés préférés sont ceux où le groupe (C₁-C₄)Alk est un groupe méthyle ou éthyle.

D'autres composés préférés sont ceux où R₂ est choisi parmi -COOH, -COO(C₁-C₄)Alk, -CN, -NO₂, -CONR₃R₄, - NHSO₂-(C₁-C₄)Alk.

D'autres composés préférés sont ceux où R₂ est un halogène notamment le chlore.

D'autres composés préférés encore sont ceux où n et m sont zéro.

Les composés de formule (I) ou (Ia) peuvent être préparés en traitant un composé de formule (II) : 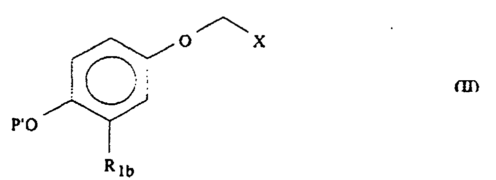 dans laquelle R₁b est R₁ₐ ou R₁ tels qu'indiqués ci-dessus, P' est un groupe protecteur et X est un groupe de formule (a) ou (b) 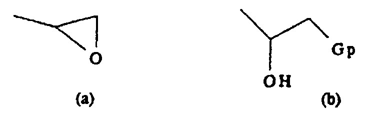 où Gp est un groupe partant tel que le tosylate, le mésylate ou un halogène, avec une amine de formule (III) 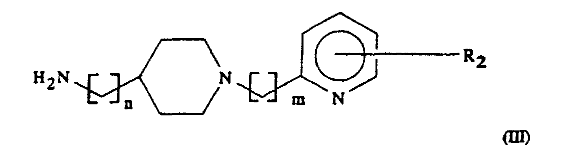 où n, m et R₂ sont tels que définis ci-dessus, en clivant le groupe P' selon les méthodes usuelles et éventuellement transformant le composé de formule (I) ou (Ia) ainsi obtenu en un de ses sels.

Plus particulièrement, la réaction entre les composés de formule (II) et (III) est réalisée dans un solvant organique, tel qu'un alcool inférieur comme le méthanol, l'éthanol et l'isopropanol; le diméthylsulfoxyde; un éther linéaire ou cyclique; un amide comme le diméthylformamide ou le diméthylacétamide; en utilisant des quantités au moins équimoléculaires des réactifs, éventuellement en faible excès d'amine.

La température de la réaction est comprise entre la température ambiante et la température de reflux du solvant choisi.

Comme groupes protecteurs P', on peut utiliser les groupes protecteurs usuels pour les groupes hydroxy tels que par exemple le méthoxyéthoxyméthyle (MEM) ou le benzyle.

Le clivage de ces groupes protecteurs est effectué selon les méthodes habituelles pour le groupe protecteur choisi; dans le cas du groupe benzyle par exemple par hydrogénation en présence d'un catalyseur tel que le Pd/C dans un solvant convenable; dans le cas du méthoxyéthoxyméthyle (MEM) on peut par contre utiliser un acide tel que l'acide trifluoroacétique.

La plupart des époxydes de formule (II) sont des composés connus en littérature ou bien ils peuvent être préparés par des procédés analogues à ceux décrits dans la littérature.

Certains époxydes de formule (II) sont par exemple décrits dans WO 96/04233 et dans US 4,396,629.

Les isomères purs des composés de formule (II) où X est un groupe (a) et R_{1b} représente le groupe SOCH₃, résolus au carbone asymétrique, sont nouveaux, ayant été obtenus pour la prémière fois comme stéréoisomères purs dépourvus d'autres stéréroisomères et d'autres impuretés.

Certaines époxydes de formule (II) sont nouveaux et constituent un autre objet de la présente invention.

Plus particulièrement, il s'agit des composés de formule (II'): 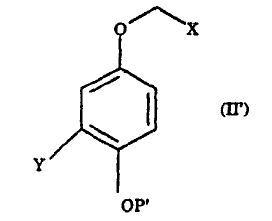 dans laquelle P' est tel que défini dans la formule (II), X est un groupe (a) tel que défini ci-dessus et Y représente un atome de brome ou un groupe 2-furyle, leurs isomères optiquement actifs et leurs sels. Ils sont préparés comme décrits dans les exemples 44 et 47.

Les amines de formule (III) peuvent être préparées par réaction des pyridines convenables de formule (IV) 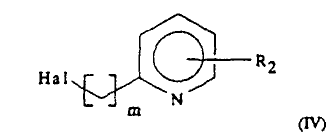 où Hal représente un halogène et R₂ et m sont tels que définis ci-dessus, avec une pipéridine de formule (V) ci-dessous 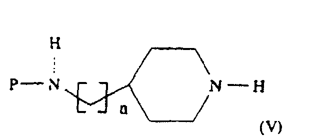 où n est tel que défini ci-dessus et P représente un groupe protecteur, dans un solvant organique en présence d'une base, suivie par clivage du groupe P des composés de formule (VI) ainsi obtenus.

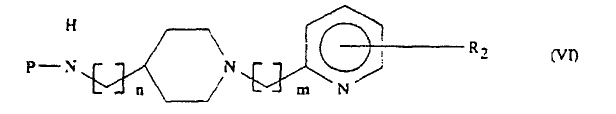

Comme solvant de réaction, on peut bien utiliser par exemple le diméthylformamide, la pyridine, le diméthylsulfoxyde, un éther linéaire ou cyclique ou un solvant chloruré tel que le dichlorométhane.

Comme base on peut utiliser par exemple un hydroxyde alcalin, un carbonate alcalin tel que le carbonate de potassium ou une amine tertiaire telle que la triéthylamine.

La réaction de condensation ci-dessus est complétée en quelques heures, normalement en 2-12 heures.

La température de réaction est comprise entre la température ambiante et la température de reflux du solvant choisi.

Comme groupes protecteurs P, on peut utiliser les groupes protecteurs usuels pour les amines tels que par exemple le *tert*-butoxycarbonyle, l'acétyle, le carbobenzyloxy.

Le clivage de ces groupes protecteurs est effectué selon les méthodes habituelles décrites le groupe protecteur choisi; dans le cas du *tert*-butoxycarbonyle par exemple, le clivage est normalement effectué par hydrolyse acide.

Certaines des amines intermédiaires de formule (III) et (VI), regroupées dans la formule (VII) ci-dessous 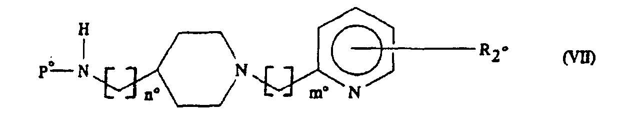 où
P° est l'hydrogène ou un groupe protecteur;
n° et m° sont 0, 1 ou 2;
R₂° est un (C₁-C₄)Alk ; (C₁-C₄)alcoxyle, -COOH, -COO(C₁-C₄)Alk , -CN, - NO₂, -CONR₃°R₄°, -SO₂NH₂, -NHSO₂(C₁-C₄)Alk ;
R₃° et R₄° sont l'hydrogène ou un groupe (C₁-C₄)Alk;
à la condition que lorsque n° et m° sont zéro, R₂° est autre que méthoxy dans la position 6 et qu'halogène dans la position 3 ou 6 de la pyridine; lorsque n° est 1 m° est 0, R₂° est autre que chlore dans la position 6 de la pyridine et lorsque n° est 0 m° est 0 ou 1, R₂° est autre que méthyle;
ainsi que leurs sels, sont des composés nouveaux et constituent un objet ultérieur de la présente invention.

Des composés de formule (VII) particulièrement préférés sont ceux où R₂° est choisi parmi les groupes -COOH, -COO(C₁-C₄)Alk, -CN, NO₂, -CONR₃°R₄°, -SO₂NH₂. et
-NHSO₂(C₁-C₄)Alk.

D'autres composés de formule (VII) préférés sont ceux où R₂° est dans la position 5 ou 6 de la pyridine.

D'autres composés de formule (VII) préférés sont ceux où n° et m° sont zéro.

D'autres composés de formule (VII) particulièrement préférés sont ceux où P° est l'hydrogène.

Les composés de formule (I) et (Ia) ont montré une affinité très puissante vis-à-vis des récepteurs β₃.

L'activité des composés de la présente invention vis-à-vis de l'activité β₃ a été mise en évidence à l'aide d'essais in vitro sur le colon humain selon la méthode décrite dans EP-B-436435 et dans T. Croci et al, Br. J. Pharmacol., 1997, 122: 139P.

Plus particulièrement, on a constaté que les composés de formule (I) et (Ia) sont beaucoup plus actifs sur le côlon isolé que sur l'oreillette et sur la trachée.

Ces propriétés surprenantes des composés de formule (I) et (Ia) permettent d'envisager leur utilisation comme médicaments à action β₃.

De plus, les composés de formule (I) et (Ia) sont peu toxiques; notamment, leur toxicité aigüe est compatible avec leur utilisation comme médicaments pour le traitement de maladies dans lesquelles les composés ayant une affinité pour le récepteur β₃ trouvent leur application. Les composés de formule (I) et (Ia), ainsi que leurs sels pharmaceutiquement acceptables, peuvent donc être indiqués par exemple dans le traitement des maladies gastro-intestinales telles que le syndrôme du colon irritable, comme modulateurs de la motricité intestinale, comme lipolytiques, agents anti-obésité, anti-diabétiques, psychotropes, anti-glaucomateux, cicatrisants, anti-dépressants, comme inhibiteur des contractions utérines, comme tocolytiques pour prévenir ou retarder les accouchement précoces, pour le traitement et/ou la prophylaxie de la dysménorrhée.

L'utilisation des composés de formule (I) et (Ia) ci dessus, ainsi que celle de leurs sels et solvates pharmaceutiquement acceptables pour la préparation de médicaments ci-dessus, constitue un aspect ultérieur de la présente invention.

Pour une telle utilisation, on administre aux mammifères qui nécessitent un tel traitement une quantité efficace d'un composé de formule (I) ou (Ia) ou d'un de ses sels et solvates pharmaceutiquement acceptables.

Les composés de formule (I) et (Ia) ci-dessus et leurs sels et solvates pharmaceutiquement acceptables peuvent être utilisés à des doses journalières de 0,01 à 20 mg par kilo de poids corporel du mammifère à traiter, de préférence à des doses journalières de 0,1 à 10 mg/kg. Chez l'être humain, la dose peut varier de préférence de 0,5 mg à 1500 mg par jour, notamment de 2,5 à 500 mg selon l'âge du sujet à traiter, le type de traitement, prophylactique ou curatif, et la gravité de l'affection. Les composés de formule (I) et (Ia) sont généralement administrés en unité de dosage de 0,1 à 500 mg, de préférence de 0,5 à 100 mg de principe actif, une à cinq fois par jour.

Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I) ou (Ia) ci-dessus ou un de ses sels et solvates pharmaceutiquement acceptables.

Dans les compositions pharmaceutiques de la présente invention pour l'administration orale, sublinguale, sous-cutanée, intramusculaire, intraveineuse, topique, transdermique ou rectale, les ingrédients actifs de formule (I) ou (Ia) ci-dessus, leurs sels et solvates pharmaceutiquement acceptables peuvent être administrés sous formes unitaires d'administration, en mélange avec des supports pharmaceutiques classiques, aux animaux et aux êtres humains, pour le traitement des affections susdites. Les formes unitaires d'administration appropriées comprennent les formes par voie orale telles que les comprimés, les gélules, les poudres, les granulés et les solutions ou suspensions orales, les formes d'administration sublinguale et buccale, les formes d'administration sous-cutanée, intramusculaire ou intraveineuse, les formes d'administration locale et les formes d'administration rectale.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granulés dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration locale, on mélange le principe actif dans un excipient pour la préparation de crèmes ou onguents ou on le dissout dans un véhicule pour l'administration intraoculaire, par exemple sous forme de collyre.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Selon un autre de ses aspects, la présente invention concerne une méthode de traitement des pathologies qui sont améliorées par une action β₃-agoniste, qui comprend administrer un composé de formule (I) ou (Ia) ou l'un de ses sels ou solvates pharmaceutiquement acceptables.

Les composés de formule (I) et (Ia), notamment les composés (I) et (Ia) marquées par un isotope, peuvent aussi être utilisés comme outils de laboratoire dans des essais biochimiques.

Les composés de formule (I) et (Ia) se lient au récepteur β₃-adrénergique. On βpeut donc utiliser ces composés dans un essai ordinaire de liaison ("binding"), dans lequel on emploie un tissu organique où ce récepteur est particulièrement abondant, et on mesure la quantité de composé (I) ou (Ia) déplacé par un composé test, pour évaluer l'affinité dudit composé vis-à-vis des sites de liaison de ce récepteur particulier.

Un autre objet spécifique de la présente invention est donc un réactif utilisable dans les essais biochimiques, qui comprend au moins un composé de formule (I) ou (Ia) convenablement marqué.

Les exemples qui suivent illustrent mieux l'invention.

### PREPARATION 1

### 4-tert-butoxycarbonylamino-pipéridine.

On mélange à la température ambiante pendant 2 heures 25 g (0,13 mole) de 4-amino-1-benzylpipéridine, 36,2 ml (0,26 mole) de triéthylamine et 31,2 g (0,143 mole) de di-*tert*-butyl-dicarbonate dans 200 ml de diméthylformamide. On verse le mélange dans de l'eau, on extrait à l'acétate d'éthyle, on lave à l'eau et on cristallise le produit ainsi obtenu dans 200 ml d'éther isopropylique. On obtient 33 g de 1-benzyl-4-*tert*-butoxycarbonylamino-pipéridine qu'on hydrogène dans un mélange de 200 ml d'éthanol et 100 ml de tétrahydrofurane en présence de 3 g de Pd/C à 10%. Après filtration du catalyseur, on isole le composé du titre. P.f. 157-160°C.

### PREPARATION 2

### 4-tert-butoxycarbonylaminométhyl-pipéridine.

En opérant comme décrit dans la préparation 1, mais en utilisant la 4-aminométhyl-1-benzylamine au lieu de la 4-amino-1-benzylamine, on obtient le composé du titre. P.f. 105-107°C.

### EXEMPLE 1

### 4-tert-butoxycarbonylamino-1-(5-aminocarbonylpyridi-2-yl)-pipéridine.

On chauffe à 80°C pendant 18 heures un mélange de 3 g (0,015mole) du produit de la préparation 1, 1,5 g (0,015 mole) de triéthylamine et 2,34 g (0,015 mole) de 6-chloronicotinamide dans 60 ml de diméthylformamide. Après refroidissement on ajoute de l'eau et on filtre le produit. On obtient ainsi 2,8 g du composé du titre. P.f. 255°C déc.

### EXEMPLE 2

### 4-amino-1-(5-aminocarbonylpyrid-2-yl)-pipéridine et son dichlorhydrate hémihydraté.

On mélange 1,84 g (0,0057 mole) du produit de l'exemple 1 et 50 ml d'acétate d'éthyle.

On y ajoute 50 ml d'une solution 3N d'acide chlorhydrique dans de l'acétate d'éthyle sous agitation et on laisse agiter à la température ambiante pendant 10 heures. On filtre et on lave à l'acétone. On obtient ainsi 1,67 g du composé du titre. P.f. 290°C déc.

### EXEMPLE 3

### 4-tert-butoxycarbonylamino-1-(5-éthoxycarbonylpyrid-2-yl)-pipéridine.

On fait réagir un mélange comme décrit dans l'exemple 1 mais en utilisant l'ester éthylique de l'acide 6-chloronicotinique au lieu du 6-chloronicotinamide. Après refroidissement, on y ajoute de l'eau, on extrait l'acétate d'éthyle, on sèche la phase organique sur du sulfate de sodium et on évapore le solvant sous pression réduite. On obtient le composé du titre. P.f. 140-142°C.

### EXEMPLE 4

### 4-amino-1-(5-éthoxycarbonylpyrid-2-yl)-pipéridine et son dichlorhydrate hydraté.

En opérant comme décrit dans l'exemple 2 mais en utilisant le produit de l'exemple 3 au lieu du produit de l'exemple 1, on obtient le composé du titre. P.f. 148-150°C.

### EXEMPLE 5

### 4-tert-butoxycarbonylamino-1-(5-nitropyrid-2-yl)-pipéridine.

En opérant comme décrit dans l'exemple 1 mais en utilisant la 2-chloro-5-nitropyridine au lieu du 6-chloronicotinamide, on obtient le composé du titre. P.f. 220°C.

### EXEMPLE 6

### 4-amino-1-(5-nitropyrid-2-yl)-pipéridine.

En opérant comme décrit dans l'exemple 2 mais en utilisant le produit de l'exemple 5 au lieu du produit de l'exemple 1, on obtient le chlorhydrate composé du titre. P.f. 266-270°C.

On libère la base à l'aide d'une solution de H₂O/NH₄OH et extraction à l'acétate d'éthyle. On obtient le composé du titre. P.f. 115-117°C.

### EXEMPLE 7

### 4-tert-butoxycarbonylamino-1-(5-cyanopyrid-2-yl)-pipéridine.

En opérant comme décrit dans l'exemple 1 mais en utilisant la 2-chloro-5-cyanopyridine au lieu du 6-chloronicotinamide, on obtient le composé du titre. P.f. 192-194°C.

### EXEMPLE 8

### 4-amino-1-(5-cyanopyrid-2-yl)-pipéridine.

En opérant comme décrit dans l'exemple 2 mais en utilisant le produit de l'exemple 7 au lieu du produit de l'exemple 1, et en traitant le sel chlorhydrate ainsi obtenu avec une solution aqueuse de NH₄OH, on obtient le composé du titre. P.f. 68-72°C.

### EXEMPLE 9

### 9a/ 3-[1-(5-aminocarbonylpyrid-2-yl)-4-pipéridinylamino]-1-(4-benzyloxyphénoxy)-2-propanol.

On mélange 1,2 g (0,0054 mole) du produit de l'exemple 2 (base), 1,4 g (0,0054 mole) de 4-benzyloxy-1-(2,3-époxypropoxy)benzène dans 50 ml de diméthylsulfoxyde et on chauffe à 70°C pendant 18 heures. On verse dans de l'eau, on extrait à l'acétate d'éthyle, on sèche, on filtre et on évapore le solvant sous pression réduite. On obtient le composé du titre. P.f. 160-162°C.

### 9b/ 3-[1-(5-aminocarbonylpyrid-2-yl)-4-pipéridinylamino]-1-(4-hydroxy phénoxy)-2-propanol.

On hydrogène 0,77 g du composé de l'étape précédente à 40 °C à la pression ambiante dans 30 ml de THF et 30 ml d'éthanol en présence de 0,1 g de Pd/C à 10% pendant 6 heures environ. On filtre le catalyseur et on évapore sous pression réduite. On purifie le brut de réaction par flash-chromatographie en éluant par un mélange chlorure de méthylène/méthanol = 8/2. On obtient le composé du titre. P.f. 192-194°C

### EXEMPLE 10

### 3-[1-(5-éthoxycarbonylpyrid-2-yl)-4-pipéridinylamino]-1-(4-hydroxyphénoxy)-2-propanol.

En opérant comme décrit dans l'exemple 9 mais en utilisant le produit de l'exemple 4 au lieu du produit de l'exemple 2, on obtient le composé du titre. P.f. 157-159°C

### EXEMPLE 11

### 3-[1-(5-cyanopyrid-2-yl)-4-pipéridinylamino]-1-(4-hydroxyphénoxy)-2-propanol.

En opérant comme décrit dans l'exemple 9 mais en utilisant le produit de l'exemple 8 au lieu du produit de l'exemple 2, on obtient le composé du titre. P.f. 160-162°C.

### EXEMPLE 12

### 3-[1-(5-nitropyrid-2-yl)-4-pipéridinylamino)-1-(4-hydroxyphénoxy)-2-propanol.

### 12a/ 3-[1-(5-nitropyrid-2-yl)-4-pipéridinylamino]-1-4-méthoxy-éthoxy-méthoxy phénoxy)-2-propanol.

On chauffe au reflux pendent 17 heures un mélange de 1,06 g (0,0048 mole) du produit de l'exemple 6 et de 1,21 g (0,0048 mole) de 4-(méthoxy-éthoxy-méthoxy)-1-(2,3-époxypropoxy)-benzène dans 40 ml d'éthanol. On filtre et on cristallise le produit dans 25 ml d'éthanol absolu. On obtient ainsi le composé du titre. P.f. 122-124°C.

### 12b/ 3-[1-(5-nitropyrid-2-yl)-4-pipéridinylamino]-1-(4-hydroxyphénoxy)-2-propanol.

A une solution de 1,2 g (0,0025 mole) du produit de l'étape précédente dans 10 ml de chlorure de méthylène, on ajoute 2 ml de CF₃COOH dans 5 ml de chlorure de méthylène. On agite à la température ambiante pendant 48 heures. On verse le mélange dans de l'eau, on y ajoute une solution de H₂O/NH₄OH jusqu'à un pH basique, on extrait à l'acétate d'éthyle et on évapore le solvant sous pression réduite. On purifie par flash-chromatographie (éluant: chlorure de méthylène/méthanol = 9/1. On obtient le composé du titre. P.f. 195-197°C.

### EXEMPLE 13

### 4-tert-butoxycarbonylamino-1-(4-éthoxycarbonylpyrid-2-yl)-pipéridine.

En opérant comme décrit dans l'exemple 3 mais en utilisant l'ester éthylique de l'acide 2-chloro-4-pyridincarboxylique au lieu de l'ester éthylique de l'acide 6-chloronicotinique, on obtient le composé du titre. P.f. 105-107°C.

### EXEMPLE 14

### 4-amino-1-(4-éthoxycarbonylpyrid-2-yl)-pipéridine et son dichlorhydrate hydraté.

En opérant comme décrit dans l'exemple 2 mais en utilisant le produit de l'exemple 13 au lieu du produit de l'exemple 1, on obtient le composé du titre. P.f. 222-224°C déc.

### EXEMPLE 15

### 3-[1-(4-éthoxycarbonylpyrid-2-yl)-4-pipéridinylamino]-1-(4-hydroxyphénoxy)-2-propanol.

En opérant comme décrit dans l'exemple 9 mais en utilisant le produit de l'exemple 14 (base), au lieu du produit de l'exemple 2, on obtient le composé du titre. P.f. 115-117 °C.

### EXEMPLE 16

### 4-tert-butoxycarbonylamino-1-(pyrid-2-ylméthyl)-pipéridine.

En opérant comme décrit dans l'exemple 3 mais en utilisant la 2-chlorométhylpyridine au lieu de l'ester éthylique de l'acide 6-chloronicotinique, on obtient le composé du titre. P.f. 98-100°C.

### EXEMPLE 17

### 4-amino-1-(pyrid-2-yl-méthyl)-pipéridine et son chlorhydrate dihydraté.

En opérant comme décrit dans l'exemple 2 mais en utilisant le produit de l'exemple 16 au lieu du produit de l'exemple 1, on obtient le composé du titre. P.f. 270-273°C.

### EXEMPLE 18

### 3-[1-(pyrid-2-yl-méthyl)-4-pipéridinylamino]-1-(4-hydroxy-phénoxy)-2-propanol.

En opérant comme décrit dans l'exemple 9 mais en utilisant le produit de l'exemple 17 base au lieu du produit de l'exemple 2, on obtient le composé du titre. P.f. :190-192°C.

### EXEMPLE 19

### 4-tert-butoxycarbonylaminométhyl-1-(5-éthoxycarbonylpyrid-2-yl)-pipéridine.

En opérant comme décrit dans l'exemple 3 mais en utilisant le produit de la préparation 2 au lieu du produit de la préparation 1, on obtient le composé du titre. P.f. 118-120°C.

### EXEMPLE 20

### 4-aminométhyl-1-(5-éthoxycarbonylpyrid-2-yl)-pipéridine et son dichlorhydrate.

En opérant comme décrit dans l'exemple 2 mais en utilisant mais en utilisant le produit de l'exemple 19 au lieu du produit de l'exemple 1, on obtient le composé du titre. P.f. 230-232°C.

### EXEMPLE 21

### 3-{[1-(5-éthoxycarbonylpyrid-2-yl)-4-pipéridinylméthyl]amino}-1-(4-hydroxyphénoxy)-2-propanol (chlorhydrate).

En opérant comme décrit dans l'exemple 9 mais en utilisant le produit de l'exemple 20 (base) au lieu du produit de l'exemple 2, on obtient le composé du titre base. On prépare le chlohydrate à l'aide d'une solution d'acide chlorhydrique en isopropanol. On obtient le composé du titre. P.f. 195°C déc.

### EXEMPLE 22

### 4-tert-butoxycarbonylamino-1-(pyrid-2-yl)-piperidine.

En opérant comme décrit dans l'exemple 3 mais en utilisant la 2-bromo-pyridine au lieu de l'ester éthylique de l'acide 6-chloronicotinique on obtient le composé du titre. P.f. : 118-120°C.

### EXEMPLE 23

### 4-amino-1-(pyrid-2-yl)-pipéridine et son chlorohydrate.

En opérant comme décrit dans l'exemple 2 mais en utilisant le produit issu de l'exemple 22 on obtient le composé du titre. P.f. : 227-230°C.

### EXEMPLE 24

### 3-[1-(pyrid-2-yl)-4-pipéridinylamino]-1-(4-hydroxy-phénoxy)-2-propanol et son dichlorhydrate hydraté.

En opérant comme décrit dans l'exemple 9 mais en utilisant le produit de l'exemple 23 base au lieu du produit de l'exemple 2 et de l'éthanol absolu au lieu du diméthylsulfoxide on obtient le composé du titre base. On prépare le chlorydrate à l'aide d'une solution d'acide chlorhydrique en isopropanol. On obtient le composé du titre. P.f.: 137-139.

### EXEMPLE 25

### (2S)-3-[1-(5-éthoxycarbonylpyrid-2-yl)-4-pipéridinylamino]-1-(4-hydroxyphénoxy)-2-propanol.

### 25a) (2S)-3-[1-(5-éthoxycarbonylpirid-2-yl)-4-pipéridinylamino]-1-(4-benzyloxy phénoxy)-2-propanol.

On mélange 0,81g (0,0033 mole) du produit de l'exemple 4 avec 0,83g (0,0033 mole) de (2S)-4-benzyloxy-1-(2,3-époxypropoxy)benzène dans 90 ml de éthanol et on chauffe au reflux pendant 20 heures. On évapore le solvant et on ajoute de l'éther éthylique. On filtre et on purifie le brut de réaction par chromatographie en éluant par un mélange chlorure de méthylène/méthanol = 95/5.

On obtient le composé du titre. P.f. : 105-107°C; [α_{D}] = -5,12° (C = 1% en CHCl₃).

### 25b) (2S)-3-[1-(5-éthoxycarbonylpyrid-2-yl)-4-pipéridinylamino]-1-(4-hydroxyphénoxy)-2-propanol.

Le produit issu de l'étape précédente est soumis à une hydrogénation selon la procédure décrite dans l'exemple 9b. Le brut de réaction est purifié par flash-chromatographie en éluant par un mélange chlorure de méthylène/méthanol = 95:5. On obtient le composé du titre. P.f.. 135-137 °C, [α_{D}] = 5,64 (C=1% en CH₃OH).

### EXEMPLE 26

### 26a) 3-[1-(5-éthoxycarbonylpyrid-2-yl)-4-pipéridinylamino]-1-[(4-benzyloxy-3-méthylsulfinyl)-phénoxy]-2-propanol.

On mélange 0,75 g (0,0024 mole) de 4-benzyloxy-3-méthylsulfinyl-1-(2,3 époxypropoxy)benzène (préparé selon le procédé décrit dans US 4,396,629) avec 0,646 g (0,0026 mole) du produit de l'exemple 4 base dans éthanol absolu. On chauffe au reflux pendant 4 heures, on sèche et on purifie le brut de réaction par chromatographie en éluant avec des mélanges de CH₂Cl₂, CH₃OH, NH₄OHconc en proportion variables entre 95 :5 :0,2 et 90 :10 :1. On obtient le produit de réaction. P.f. : 113-130 °C.

### 26b) 3-[1-(5-éthoxycarbonylpyrid-2-yl)-4-pipéridinylamino]-1-[(4-hydroxy-3-méthylsulfinyl)-phenoxy]-2-propanol et son dichlorohydrate dihydraté

On chauffe le produit de l'étape précédente pendant 7 heures à 55°C dans 20 ml de CF₃COOH. On évapore partiellement le solvant, on ajoute de l'acétate d'éthyle et du toluène et on évapore encore le solvant. On ajoute de l'eau sature en Na₂CO₃ et on extrait à l'acétate d'éthyle. Le brut de réaction est séché et purifié par flash-chromatographie en éluant par un mélange de CH₂Cl₂, CH₃OH, NH₄OHconc en proportions variables entre 92 :8 :0,8 et 90:10 :1. On obtient le composé du titre comme base libre (P.f. : 95-100°C). On dissout le produit dans l'acétone et on ajoute une solution de HCl en isopropanol, on filtre et on lave à l'acétone. On obtient le produit du titre. P.f. :173-175°C.

### EXEMPLE 27

### 4-tert butoxycarbonylamino-1-(5-méthylpyrid-2-yl)pipéridine.

On chauffe à 130°C pendant une nuit 2,03g (0,0101 mole) du produit de la préparation 1 avec 3,5 g (0,0203 mole) de 2-bromo-5-méthylpyridine et 1,5 ml de triéthylamine dans 50 ml de diméthylsulfoxide. On verse dans l'eau, on extrait le brut de réaction qui est purifié par flash-chromatographie (cyclohéxane/éthyleacétate = 1/3). P.f. : 121-123°C.

### EXEMPLE 28

### 4-amino-1-(5-méthylpyrid-2-yl)-pipéridine et son dichlorhydrate hydraté.

On chauffe au reflux pendant 4 heures une solution contenant le produit de l'exemple 27 dans 18 ml de éthyle acétate et 18 ml de HCl en éthyle acétate (environ 3N). On filtre et on lave à l'acétone. On cristallise en isopropanol et on obtient le composé du titre. P.f.: 281-283 °C.

### EXEMPLE 29

### 29a) 3-[1-(5-méthylpyrid-2-yl)-4-pipéridinylamino)-1-(4-benzyloxyphénoxy)-2-propanol.

On chauffe au reflux pendant une nuit 1,81 g (0,00956 mole) du produit de l'exemple 28 et 2,2 g (0,00860 mole)de 4-benzyloxy-1-(2,3-époxyproxy)-benzène dans 100 ml d'éthanol absolu. On évapore le solvant et on purifie le brut de réaction par flash-chromatographie en éluant par un mélange cyclohéxane/éthyle acétate = 6/4 et par le suite métahnol/ammoniac = 100/1. On obtient le produit du titre. P.f. 123-125°C.

### 29b) 3-[1-(5-méthylpyrid-2-yl)-4-pipéridinylamino]-1-(4-hydroxyphénoxy)-2-propanol et son dichlorydrate.

On opère comme décrit en 29b mais on utilise le produit de l'exemple 29a. Le brut de réaction est purifié par flash-chromatographie en éluant par un mélange éthyl acétate/méthanol = 4.6. On obtient le composé du titre comme base. On prépare le chlorhydrate par addition de HCl en isopropanol. P.f. : 151-153 °C.

### EXEMPLE 30

### 4-tert-butoxycarbonylamino-1-(5-méthoxycarbonyl-pyrid-2-ylméthyl)-piperidine

On chauffe à 40 °C pendant 2 heures une solution contenant 0,7 g (0,0034 mole) du produit de la préparation 1, 0,53 g (0,0023 mole) de 2-bromométhyl-5-méthoxycarbonyl-pyridine (préparée selon la procédure décrite en J. Med. Chem., 1992, 3, 490-501), 0,26 ml (0,0025 mole) de triéthylamine dans 10 ml de diméthylformamide. On verse dans l'eau, on extrait à l'acétate d'éthyle et on purifie le brut de réaction par flash-chromatographie (éluant acétate d'éthyle). On obtient le composé du titre. P.f. : 110-112 °C.

### EXEMPLE 31

### 4-amino-1-(5-méthoxycarbonyl-pyrid-2-ylméthyl)-pipéridine

Le produit de l'exemple 30 est chauffé pendant 3 heures à 40 °C dans une solution de CH₂Cl₂ contenant 11 ml de CF₃ COOH. On évapore le solvant et on cristallise en acétone. On obtient le produit du titre. P.f.: 173°-175°C.

### EXEMPLE 32

### 3-[1-(5-méthoxycarbonyl-pyrid-2-ylméthyl)-4-pipéridinylamino]-1-(4-hydroxy phénoxy)-2-propanol

On opérant comme décrit dans l'exemple 12 mais en utilisant le produit de l'exemple 31 au lieu du produit de l'exemple 6 on obtient le produit du titre. P.f. : 141-143 °C.

### EXEMPLE 33

### 4-tert-butoxycarbonylaminométhyl-1-(5-méthoxycarbonyl-pyrid-2-ylméthyl)-piperidine

On chauffe pendant deux heures dans 25 ml de diméthylformammide 1.68 g (0,0078 mole) du produit de la préparation 2, 1 ml (0,0068 mole) de triéthylamine et 1,5 g (0,0065 mole) de 2-bromométhyl-5-méthoxycarbonyl-pyridine. On verse dans l'eau, on extrait à l'acétate d'éthyle, on lave à l'eau et on sèche le produit. On cristallise en éther isoprophylique. P.f. : 103-105°C.

### EXEMPLE 34

### 4-aminométhyl-1-(5-méthoxycarbonyl-pyrid-2-ylméthyl)-pipéridine trifuorocétate

On chauffe à 40 °C pendant 3 heures une solution contenant 1,7 g (0,0046 mole) du produit de l'exemple 33, 15 ml de CF₃COOH et 15 ml de CH₂Cl₂. On évapore le solvant sous vide, on ajoute de l'ammoniac concentré et on extrait à l'éthyl acétate. On évapore le solvant et on obtient le produit du titre. P.f. : 193-195 °C.

### EXEMPLE 35

### 3-[1-(5-méthoxycarbonyl-pyrid-2-ylméthyl)-4-pipéridinylaminométhyl]-1-(4-hydroxyphénoxy)-2-propanol

En opérant comme décrit dans l'exemple 9 mais en utilisant le produit de l'exemple 34 au lieu du produit de l'exemple 2 on obtient le composé du titre.

### EXEMPLE 36

### (2R)-3-[5-éthoxycarbonylpyrid-2-yl)-4-pipéridinylamino]-1-(4-hydroxy-phénoxy)-2-propanol.

En opérant comme décrit dans l'exemple 25 mais en utilisant la (2R)-4-benzyloxy-1-(2,3-époxypropoxy)benzène au lieu de la (2S)-4-benzyloxy-1-(2,3-époxypropoxy)benzène on obtient le composé du titre. P.f. : 134°-135°C. [α]_{D}= +4,2° (C= 1% en CH₃OH)

### EXEMPLE 37

### (2S)-4-benzyloxy-3-méthylsulfinyl-1-(2,3-époxypropropoxy)benzène.

On dissout sous agitation et atmosphère d'azote 0,71 g de NaH au 60% (0,0177 mole) dans 15 ml de dimethylformamide on ajoute lentement (pendant 30 minutes) 4,45 g (0,0169 mole) de 4-benzyloxy-3-méthylsulfinylphénol (préparé selon le procédé décrit dans US 4,396,629) dans 35 ml de dimethylformamide et après, 4,40 g (0,0169 mole) de S(+)glycildylnosilate dans 10 ml de dimethylformamide. Après l'introduction du dernier réactif le mélange est laissé réagir à température ambiante pendant 3 heures. On ajoute de l'eau, on extrait à l'acétate d'éthyle et on évapore le solvant. On purifie le brut de réaction par flash-chromatographie en eluant par un mélange CH₂Cl2:MeOH = 97/3. [α]₃₆₅ₙₘ = - 14,3°; [α]₄₃₆ₙₘ = -3,9° (C=1% en CH₃OH, t = 20°C). On obtient le composé du titre.

Le produit ainsi obtenu est soumis à une analyse HPLC aux conditions suivantes :
- phase stationnaire chirale :CHIRALCEL OD-H
- phase mobile : hexane/éthanol = 80/20 (0.5 ml/min).

On observe deux pics et TR₁ = 20,780 min. et TR₂ =23.900 min. correspondant aux diastéréoisomères ayant différente configuration à l'atome de soufre.

### EXEMPLE 38

### 38a) (2S) 3-[1-(5-éthoxycarbonylpyrid-2-yl)-4-pipéridinylamino-1-[(4-benzyloxy-3-méthylsulfinyl)-phenoxy]-2-propanol

On chauffe au reflux pendant une nuit 4.65 g (0.0146 mole) du produit de l'exemple 37 et 3,82 g (0.0153 mole) du produit de l'exemple 4 sous forme de base dans 60 ml d'éthanol. On évapore le solvant et on purifie le brut de réaction par flash-chromatographie en éluant par un mélange CH₂Cl₂/méthanol = 97/3. On obtient le produit du titre. P.f. : 109-110°C

Le produit ainsi obtenu est soumis à une analyse HPLC aux conditions suivantes :
- phase stationnaire chirale : CHIRALCEL OD-H
- phase mobile : hexane/éthanol = 65/35 - (0,6 ml/min, 660 psi)

On observe deux pics, TR₁ = 20,687 min. et TR₂ =28,327 min. correspondant aux diastéréoisomères ayant différente configuration à l'atome de soufre.

### 38b) (2S) 3-[1-(5-éthoxycarbonylpyrid-2-yl)-4-pipéridinylamino-1-[(4-hydroxy-3-méthylsulfinyl)-phenoxy]-2-propanol et son dichlorhydrate hydraté.

Le produit obtenu dans l'exemple 38a) est traité avec 100 ml de CF₃COOH à 55°C pendant 6 heures. On évapore le solvant et on ajoute de l'eau saturée en Na₂CO₃ et de la glace.

On extrait avec de l'acétate d'éthyle on lave à l'eau en on évapore le solvant. Le brut de réaction est purifié par flash-chromatographie en éluant par un mélange CH₂Cl₂/méthanol = 9/1. On obtient le produit du titre : P. f. : 62-65°C. On prépare le dichlorhydrate hydraté à l'aide d'une solution de HCl en isopropanol. P.f. 172-174°C. [α]_{D} = +4,0°; [α]₄₃₆ₙₘ = +11,7° ; [α]₅₄₆ₙₘ = +5,1 ° ; (C = 1% en méthanol). Le produit base est soumis à une analyse HPLC aux conditions suivantes :
- phase stationnaire chirale : CHIRALCEL OD-H
- phase mobile : isopropanol (0,4 ml/min, 1880 psi)

On observe deux pics, TR₁ = 11,900 min. et TR₂ =13,713 min. correspondant aux diastéréoisomères ayant différente configuration à l'atome de soufre.

### 38C) Séparation des diastéréoisomères du 38b)

Le produit issu de l'exemple précédente a été soumis à une HPLC chirale préparative pour la séparation des diastéréoisomères dans les conditions suivantes :
Appareil : PROCHROM LC 50 à compression dynamique axiale. Diamètre de la colonne 50 mm.
   Longueur de la phase stationnaire 30 cm
Phase stationnaire : CHIRALCEL OD 390 g de phase ciralcel OD sont mis en suspension dans 200 ml de propanol et sont comprimés à 20 bars.
Phase mobile : isohexane/éthanol (91/ 9) + CF₃COOH 0,1% + triéthylamine 0,1%.

On sépare deux produits qui, après évaporation de la phase mobile, présentent un fort excès de poids en raison de la formation de sels. Ces sels ont été éliminés par purification sur phase inverse dans les conditions suivantes :
Appareil : PROCHROM LC 50
Phase stationnaire : KROMASIL C18 100Å 10µm. 380 g de phase stationnaire sont mis en suspension dans 560 ml de méthanol et sont comprimés à 40 bars.
Phase mobile : Eluant A : H₂O + CF₃COOH 0,1% / Eluant B : CH₃CN/H₂O = 90/10 + CF₃COOH 0,8%.

### Gradient d'élution :

| **t (en min)** | %A | %B |
|---|---|---|
| 0 | 90 | 10 |
| 5 | 90 | 10 |
| 50 | 67 | 33 |
| 60 | 67 | 33 |

Les deux produits purifiés ont été analysés par HPLC sur chirale aux condition suivantes :
- phase stationnaire chirale :CHIRALCEL OD
- phase mobile: isohexane/éthanol = 85/15 + CF₃COOH 0,1% + triéthylamine 0,1%.

Ces deux produits, correspondant aux diastéréoisomères ayant différente configuration à l'atome de soufre présentent des temps de rétention relative respectivement de TRR₁ = 1 et TRR₂ = 1,12. La spectrométrie de masse confirme que les deux produits obtenus ont la même masse, à savoir MH⁺= 478 et que leur fragmentation caractéristique est la même.

### EXEMPLE 39

### 4-hydroxy-3-méthylcarbonyl-1-(2,3-époxypropoxy) benzène

On chauffe au reflux pendant 8 heures une solution contenant 3,00 g (0,020 mole) de 2,5-dihydroxyacétophenone, 3,37 ml (0,040 mole) de épibromhydrine et 5,06 g (0,040 mole) de K₂CO₃ dans 30 ml de acétone. On évapore le solvant, on ajoute 20 ml d'une solution saturée en NaH₂PO₄, 120 ml d'eau et on extrait à l'acétate d'éthyle.

On évapore le solvant et on purifie le brut de réaction par chromotographie en éluant par un mélange de CH₂Cl₂ et acétate d'éthyle en rapport variable entre 100/0 et 95/5. On obtient le produit du titre. P.f. : 83-35°C.

### EXEMPLE 40

### 4-benzyloxy-3-méthylcarbonyl-1-(2,3-époxypropoxy)-benzène

On chauffe au reflux pendant 6 heures sous azote une solution contenant 1 g (0,0048 mole) du produit de l'exemple 39, 0,856 ml (0,0072 mole) de benzyl-bromure et 0,924 g (0,0072 mole) de K₂CO₃ dans 12 ml d'acétone. On ajoute encore 0,5 ml (0.0042 mole) de benzyl-bromure et on chauffe encore pendant 7 heures. On verse dans l'eau, on extrait à l'acétate d'éthyle et on évapore le solvant. Le brut de réaction est purifié par chromatographie, en éluant par un mélange héxane/acétone = 8/2. On obtient le produit du titre. P.f. : 45-48°C.

### EXEMPLE 41

### 3-[1-(5-éthoxycarbonylpyrid-2-yl)-4-pipéridinylamino]-1-[(4-hydroxy-3-méthylcarbonyl)-phenoxy]-2-propanol

En opérant comme décrit dans l'exemple 9 mais en utilisant le produit de l'exemple 40 au lieu du 4-benzyloxy-1-(2,3-époxypropoxy)-benzène on obtient le produit du titre. P.f.: 105-108°C.

### EXEMPLE 42

### (1-méthylcarbonyl-3-bromo-4-benzyloxy)-benzène

On chauffe au reflux pendant 4 heures une solution contenant 0,92 g (0,428 mole) de (2-bromo-4-méthylcarbonyl)-phénol, 51 ml (0,428 mole) de benzyl-bromure, 6,4 gr de NaI (0,043 mole) et 89,0 gr (0,642) de K₂CO₃ dans 1,5 1 d'acétone. On filtre, on évapore le solvant on ajoute de l'acétate d'éthyle et on lave à l'eau. On évapore le solvant et on obtient le produit du titre. P.f. : 117-119 °C.

### EXEMPLE 43

### (3-bromo-4-benzyloxy-1-acétoxy)-benzène

On chauffe au reflux pendant 24 heures une solution contenant 93 gr (0,3 mole) du produit de l'exemple 42 et 259 g (1,05 mole) de acide-3-chloro perbenzoique (cône. 70%) dans 2 1 de CH₂Cl₂. On évapore le solvant, on ajoute de l'acétate d'éthyle et on lave avec une solution de Na₂S₂O₅. On filtre et on évapore le solvant. On obtient le composé du titre. P.f. : 69-71°C.

### EXEMPLE 44

### [4-benzyloxy-3-(2-furyl)]phénol

On chauffe à 90°C pendant 5 heures sous atmosphère d'Argon une solution de 180 ml de dioxane contenant 13 g (0,0406 mole) du produit de l'exemple 43, 5 g (0,0447 mole) de l'acide 2-furanboronique, 1,6 g de Tetrakis (tryphénylphosphine)-Palladium et 38,3 g (0,1215 mole) de fluorure de Tetrabutylammonium. On ajoute 1 1 d'éther éthylique et on lave à l'eau.

On filtre et on évapore le solvant. Le brut de réaction est purifié par chromatographie (éluant cyclohexane/éthyle acétate = 8/29). On obtient le composé du titre. P.f. : 112-115°C.

### EXEMPLE 45

### [4-benzyloxy-3-fur-2-yl-1-(2,3-époxypropoxy)]-benzène

On laisse sous agitation à température ambiante pendant 15 minutes 8,3 g (0,031 mole) du produit de l'étape précédente avec 6,5 g (0,046 mole) de K₂CO₃ dans 60 ml de dimethylformamide. Après 15 minutes on ajoute 5,3 ml (0,062) de epibromhydrine et on chauffe à 70 °C pendant 6 heures. On verse dans un mélange d'eau et de glace et on extrait à l'acétate d'éthyle. On filtre et on évapore le solvant. On purifie le brut de réaction par chromatographie en éluant par un mélange cyclohéxane/éthyl acétate = 85/15 et par la suite cyclohéxane/éthylacétate = 9/1. On obtient le produit du titre. P.f: 62-64°C.

### EXEMPLE 46

### 3-[1-(5-éthoxycarbonylpyrid-2-yl)-4-pipéridinylamino]-1-[(4-hydroxy-3-(2-furyl)-phenoxy]-2-propanol et son dichlorydrate hydraté

On opère comme décrit dans l'exemple 26 mais en utilisant le produit de l'exemple 45 au lieu du 4-benzyloxy-3-méthylsulfinyl-1-(2,3-époxypropoxy)-benzène. Le clivage du groupe benzyle est fait par hydrogénation en acétate d'éthyle. Le brut de réaction est purifié par chromatographie en éluant par uns mélange éthyle acétate/méthanol = 85/15. On prépare le chlorhydrate dans l'isopropanol. On obtient le produit du titre. P.f. : 251-253°C déc.

### EXEMPLE 47

### 3-bromo-4-benzyloxy-phenol

On fait réagir sous agitation pendant 40 minutes à température ambiante 0,5 g (0,0155 mole) du produit de l'exemple 43 avec 18,7 ml (0,0187)de NaOH 1N dans 150 ml de méthanol. On ajoute de l'acide citrique jusqu'à pH 6 et on évapore le solvant. On ajoute de l'acétate d'éthyle, on lave à l'eau et on évapore le solvant. On obtient le composé du titre. P.f. : 61-63 °C.

### EXEMPLE 48

### 3-bromo-4-benzyloxy-1-(2,3-époxypropoxy)-benzène

On chauffe à 45 °C pendant une nuit 3,5 g (0,0125 mole) du produit de l'exemple 47, 18,8 ml (0,0188 mole) de NaOH 1M et 2,2 ml (0,025 mole) de épibromhydrine dans 50 ml de dioxane. On évapore le solvant, on ajoute de l'eau, on extrait à l'acétate d'éthyle et on évapore le solvant. On purifie le brut de réaction par chromatographie (cyclohéxane/acétate d'éthyle = 8/2). On obtient le composé du titre.

### EXEMPLE 49

### 3-[1-(5-éthoxycarbonylpyrid-2-yl)-4-pipéridinylamino]-1-[3-bromo-4-hydroxy)phenoxy]-2-propanol

On fait réagir le produit de l'exemple 48 avec le produit de l'exemple 4 (base), selon le procédé décrit dans l'exemple 9a. Le produit issu de cette réaction est traité avec du CF₃COOH à 55°C pendant 4 heures. Le brut de réaction est purifié par chromatographie (CH₂Cl₂/CH₃OH = 93/7). On cristallise le produit dans de l'isopropanol. P.f.:123-126 °C

### EXEMPLE 50

### 4-benzyloxy-3-[(N-tertbutoxycarbonyl-N-méthylsulfonyl)-amino]-1-(2,3-époxypropoxy)-benzène.

On chauffe au reflux pendant 24 heures une solution contenant 1 g (0,0025 mole) de 4-phenylméthoxy-3-[(N-tertbutoxycarbonyl-N-methansulfonyl)-amino]-phenol (préparé selon le procédé décrit dans WO 9604233), 0,75 g (0,0029 mole) de glycidylnosilate et 1,1 g (0,008 mole de K₂CO₃. On filtre, on évapore le solvant et on purifie le brut de réaction par chromatographie en éluant par un mélange cyolohexane/éthyl acétate = 7/3.

### EXEMPLE 51

### 51a) 3-[1-(5-éthoxycarbonylpyrid-2-yl)-4-pipéridinylamino]-1-[(4-benzyloxy-3-méthansulfonamido)-phenoxy]-2-propanol

On fait réagir sous agitation pendant 48 heures à température ambiante 0,77 g (0,00172 mole) du produit de l'exemple 50, 0,86 g (0,0035mole) du produit de l'exemple 4 base et 0,2 g de LiClO₄ dans 50 ml de acetonitrile. On chauffe à 40 °C pendant 9 heures et on arrête le réaction. On évapore le solvant et on ajoute 30 ml de CH₂Cl et une solution 3N de HCl en éthyle acétate (20 ml). On chauffe pendant 4 heures à 40 °C pour évaporer le solvant. On lave à l'ammoniac et on extrait à l'acétate d'éthyle. On évapore le solvant et on purifie le brut de réaction par flash chromatographie en éluant par un mélange CH₂Cl₂/CH₃OH = 9/1. On obtient le produit du titre. P.f.: 112-114°C

### 51b) 3-[1-(5-éthoxycarbonylpyrid-2yl)-4-pipéridinylamino]-1-[-4-hydroxy-3-méthansulfonamido)-phenoxy]-2-propanol

On hydrogène le produit de l'exemple 51a selon le procédé décrit dans l'exemple 9b, en opérant à une pression variable entre la pression ambiante et 50 psi. Le brut de réaction est purifié par flash-chromatographie (CH₂Cl/CH₃OH = 9/1). On obtient le produit du titre. P.f. : 77-80 °C.

### EXEMPLE 52

### 4-acétylamino-1-(5-chloropyrid-2-yl)-pipéridine

On chauffe au reflux pendant 24 heures 6,7 g (0,035 mole) de 2-bromo-5-chloropyridine 4,97 g (0,035 mole) de 4 acétylamino-piperidine et 4,83 g (0,035 mole) de K₂CO₃ dans 50 ml alcool amylique. On évapore le solvant, on ajoute de l'eau et on extrait au CH₂Cl₂.On obtient le produit du titre. P.f. : 196-198 °C.

### EXEMPLE 53

### 4-amino-1-(5-chloropyrid-2-yl)-pipéridine et son dichlorohydrate

On chauffe au reflux pendant 5 heures 5,5 g (0.022 mole) du produit de l'exemple 52 dans 25 ml de HCl 6N. On évapore le solvant, on ajoute 50 ml d'eau et une solution de NaOH jusqu'à pH basique. On extrait au CH₂Cl₂, on évapore le solvant on prépare le chlorohydrate dans de l'isopropanol, et on cristallise dans du méthanol. On obtient le produit du titre. P.f. : 295 °C déc.

### EXEMPLE 54

### 3-[1-(5-chloropyrid-2-yl)-4-pipéridinylamino]-1-(4-hydroxyphénoxy)-2-propanol

On opère comme décrit dans l'exemple 12 mais en utilisant le produit de l'exemple 53 (base) au lieu du produit de l'exemple 6. Après le traitement avec CF₃COOH on évapore, on ajoute de l'éthyle acétate et on lave à l'aide d'une solution de NaH₂PO₄ jusqu'à pH 5. On évapore le solvant et on purifie le brut de réaction par flash-chromatographie en eluant par un mélange CH₂Cl₂/méthanol = 9/1. On obtient le produit du titre. P.f : 148-150 °C.

### EXEMPLE 55

### 2-chloro-6-éthoxycarbonyl-pyridine

On chauffe au reflux pendant 8 heures 14,1 g (0,0895 mole) de 2-chloro-6-hydroxycarbonylpyridine dans 133 ml d'une solution de HCl en éthanol. On évapore le solvant, on ajoute de l'acétate d'éthyle et on lave à l'aide d'une solution de bicarbonate. On évapore encore le solvant et on purifie le brut de réaction par flash-chromatographie (cyclohéxane/acétate d'éthyle =7/3). On obtient le produit du titre.

### EXEMPLE 56

### 4-tert-butoxycarbonylamino-1-(6-éthoxycarbonylpyrid-2-yl)-pipéridine

On chauffe à 80 °C pendant 24 heures une solution contenant 1,17 g (0,0063 mole) du produit de l'exemple 55, 1,27 g (0,0063 mole) du produit de la préparation 1, 0,9 ml de triethylamine et du NaI dans 34,2 ml de dimethylformamide. On verse dans l'eau, on extrait à l'acétate d'éthyle et on évapore le solvant. Le brut de réaction est purifié par flash-chromatographie en éluant par un mélange cyclohéxane/acétate d'éthyle = 7/3.

### EXEMPLE 57

### 4- amino-1-(6-éthoxycarbonylpyrid-2-yl)-pipéridine et son chlorohydrate

On chauffe au reflux pendant 8 heures 0,99 g (0,0025 mole) du produit de l'exemple 56 dans 6 ml d'acétate d'éthyle et 6 ml d'une solution de HCl en acétate d'éthyle. On filtre, on lave à l'acétone, on ajoute de l'ammoniac et on extrait à l'acétate d'éthyle. Le brut de réaction est purifié par chromatographie en éluant par un mélange CH₃OH/NH₄OH = 100/1. On obtient le produit du titre.

### EXEMPLE 58

### 58a/ 3-[1-(6-éthoxycarbonyl-pyrid-2-yl)-4-pipéridinylamino]-1-(4-benzyloxy phénoxy)-2-propanol

On chauffe au reflux pendant une nuit 0,3 g (0,0012 mole) du produit de l'exemple 57 base, 0,292 g (0,00114 mole) de 4-benzyloxy-1-(2,3-époxypropoxy)benzène dans 15 ml d'éthanol. On évapore sous pression réduite et on purifie le brut de réaction par chromatographie en éluant par un mélange CH3OH/acétate d'éthyle 9/1.

### 58b/ 3-[1-(6-éthoxycarbonyl-pyrid-2-yl)-4-pipéridinylamino]-1-(4-hydroxyphénoxy)-2-propanol

On opérant comme décrit dans l'exemple 9b on obtient le composé du titre. P.f. : 200°C-201°C.

### EXEMPLE 59

### 59a) (2S) 3-[5-aminocarbonyl-pyrid-2-yl)-4-pipéridinylamino]-1-(4-benzyloxy-3-méthylsulfinyl-phénoxy)-2-propanol

On chauffe au reflux, sous atmosphère d'azote pendant 6 heures 1 g (0,00314 mole) du produit de l'exemple 37 et 0,83 g (0,00377 mole) du produit de l'exemple 2 sous forme de base. On évapore le solvant et on purifie le brut de réaction par chromatographie en éluant par un mélange CH₂Cl₂/méthanol/NH₄OH = 90/10/1. On obtient le produit du titre. P.f. :143-147°C.

Le produit ainsi obtenu est soumis à une analyse HPLC aux conditions suivantes:
- phase stationnaire chirale :CHIRALCEL OD-H
- phase mobile : héxane/éthanol = 20/80 - (0,4 ml/min)
   On observe deux pics, TR₁ = 17,040 min., TR₂ = 19,827 min. correspondant aux diastéréoisomères ayant different configuration à l'atome de soufre.

### 59b) (2S) 3-[5-aminocarbonyl-pyrid-2-yl)-4-pipéridinylamino]-1-(4-hydroxy-3-méthylsulfinyl-phénoxy)-2-propanol

On travaille comme décrit dans l'exemple 38b) mais en utilisant le produit da l'exemple 59a) en lieu du produit de l'exemple 38a). Le brut de réaction est purifié une première fois par chromatographie en éluant par un mélange CH₂Cl₂/méthanol/NH₄OH = 85/15/1,5 puis 80/20/2. On conduit après une deuxième chromatographie en éluant par un mélange éthyle acétate/méthanol = 80/20 suivi par un mélange CH₂Cl₂/méthanol/NH₄OH = 85/15/1,5 puis 80/20/2. On obtient le produit du titre. P.f. : 74°C.

Le produit ainsi obtenu est soumis à une analyse HPLC aux conditions suivantes:
- phase stationnaire chirale :CHIRALCEL OD-H
- phase mobile : héxane/éthanol = 65/35 - (0,6 ml/min)
   On observe deux pics, TR₁ = 13,900 min., TR₂ = 18,233 min. correspondant aux diastéréoisomères ayant different configuration à l'atome de soufre.

### EXEMPLE 60

### 3-[1-(5-hydroxycarbonyl-pyrid-2-yl)-4-pipéridinylamino]-1-[(4-hydroxy-3-méthylsulfinyl)-phenoxy]-2-propanol

On mélange à la température ambiante 0,48g (0,001 mole) du produit de l'exemple 26b 2,51 ml de NaOH 1N (0,0025 mole), 2,5 ml d'eau et 2,5 ml d'éthanol. Après 24 heures on évapore l'éthanol et on ajoute 0,144 ml de acide acétique (d=1,049, 0,0025 mole). On observe la formation d'un huile. On élimine l'eau et on ajoute 10 ml d'éthanol. On évapore le solvant et on ajoute encore de l'éthanol. On répète cette opération plusieurs fois. On obtient le produit du titre. P.f. : 215-217 déc.

### EXEMPLE 61

### (3-acétylamino-4-benzyloxy-1-acétoxy)-benzène

On dissout sous agitation 2 g (0,0077 mole) de (3-amino-4-benzyloxy-1-acétoxy)-benzène (préparé selon le procédé décrit en Synt. Commun. 22 ; 20 ; 1992 ; 2877-2882) dans 150 ml de CH₂Cl₂ on ajoute 1,3 ml de triéthylamine (0,0093 mole) et ensuite 0,8 ml de anydride acétique (0,0085 mole). Après une nuit on chauffe au reflux pendant 4 heures. On lave à l'eau, on filtre et on évapore le solvant. On ajoute du cyclohéxane et on filtre. On obtient le produit du titre. P.f. :105-107°C.

### EXEMPLE 62

### 3-acétylamino-4-benzyloxy-phénol

On dissout 3 g (0,010 mole) du produit de l'étape précédente dans 120 ml de méthanol et 30 ml d'eau. On ajoute 12 ml de NaOH 1N (0,012 mole) et on laisse sous agitation à température ambiante pendant 30 minutes. On ajoute de l'acide citrique jusqu'à pH = 6, on évapore le solvant, on ajoute de l'éthyle acétate et on lave à l'eau. On filtre et on sèche. On obtient le produit du titre.

### EXEMPLE 63

### 3-acétylamino-4-benzyloxy-1-(2,3-époxypropoxy)-benzène

On mélange sous agitation à température ambiante pendant 15 minutes 0,5 g (0,0019 mole) du produit de l'exemple 62 avec 0,4 g de K₂CO₃ ( 0,0029) dans 5 ml de diméthylformamide. Après 15 minutes on ajoute 0,32 ml (0,0038 mole) de épibromhydrine et on laisse réagir pendant une nuit. On verse dans l'eau, on extrait à l'éthyle acétate, on sèche et on filtre. Le brut de réaction est purifié par chromatographie en éluant par un mélange cyclohéxane/éthyle acétate = 6/4. On obtient le produit du titre.

### EXEMPLE 64

### 64a) 3-[1-(5-éthoxycarbonyl-pyrid-2-yl)-4-pipéridinylamino]-1-[(3-acétylamino-4-benzyloxy)-phenoxy]-2-propanol

64a) On chauffe au reflux pendant une nuit 0,22 g (0.0007 mole) du produit de l'exemple 63 avec 0,175 g (0,0007 mole) du produit de l'exemple 4 sous forme de base dans 10 ml d'éthanol. On évapore le solvant et on purifie le brut par flash-chromatographie en éluant par méthanol. On évapore le solvant et on obtient le produit du titre. P.f. : 124-126°C

### 64b) 3-[1-(5-éthoxycarbonyl-pyrid-2-yl)-4-pipéridinylamino]-1-[(3-acétylamino-4-hydroxy)-phenoxy]-2-propanol

On hydrogène le produit de l'exemple 64a selon le procédé décrit dans l'exemple 9b. On obtient le produit du titre. P.f. : 103-105°C.

### EXEMPLE 65

### (3-méthylsulfonyl-4-benzyloxy-1-phenylcarbonyloxy)-benzène

On dissout 3,26 g (0,0089 mole) de (3-méthylsulfinyl-4-benzyloxy-1-phénylcarbonyloxy)-benzène dans 30 ml d'acétone et 5 ml de méthanol et on ajoute, à une température comprise entre 5 et 10 °C, 2,86 g de monopéroxyphtalate de magnésium (MMPP) (pureté = 80%, 0,0046 mole) dissout en 20 ml de méthanol. Après 3,5 heures on ajoute encore 0,3 g (0,00048 mole) de MMPP dans 1 ml de méthanol. On laisse réagir à température ambiante pendant une nuit. On ajoute 50 ml d'eau, 5 ml d'une solution saturée en NaHCO₃ et on extrait à l'acétate d'éthyle. On évapore le solvant et on obtient le produit du titre. P.f. : 142-145°C.

### EXEMPLE 66

### 4-benzyloxy-3-méthylsulfonyl-phénol

On dissout 3,09 g (0,008 mole) du produit de l'exemple 65 dans 40 ml de THF et on ajoute 10,5 ml de NaOH 1N (0,010 mole). Après 4 heures on ajoute 25 ml d'eau et du NaH₂PO₄ jusqu'à pH 7 et on extrait à l'acétate d'éthyle. On lave avec des solutions aqueuses de NaHCO₃ et de NaCl et on évapore le solvant. On obtient le produit du titre. P.f : 154-156°C.

### EXEMPLE 67

### 4-benzyloxy-3-méthylsulfonyl-1-(2,3-époxypropoxy)-benzène.

On laisse sous agitation pendant 10 minutes 0,158 g (0,00395) de NaOH dans 10 ml de DMF et on ajoute 1 g (0,00359 mole) du phénol obtenu dans l'exemple 66. Après 10 minutes on ajoute 0,977 g (0,00377 mole) de glycidylnosilate dans 5 ml de DMF et on laisse réagir pour 24 heures à température ambiante. On ajoute 100 ml d'eau et 2 ml d'une solution de NaH₂PO₄ on extrait à l'acétate d'éthyle et on lave à l'eau. Le brut de réaction est purifié par chromathographie en éluant par un mélange CH₂Cl₂/acétate d'éthyle = 98/2 ensuite 97/3 et 96/4. On obtient le produit du titre. P.f. : 78-82°C.

### EXEMPLE 68

### 68a) 3-[1-(5-éthoxycarbonylpyrid-2-yl)-4-pipéridinamino]-1-[(4-benzyloxy-3-méthylsulfonyl)-phénoxy]-2-propanol

On chauffe au reflux pendant 6 heures 0,700 g (0.00209 mole) du produit de l'exemple 67 et 0,574 g (0,0023 mole) du produit de l'exemple 4 sous forme de base dans 6 ml d'éthanol. On évapore le solvant, on ajoute à l'huile ainsi formé de l'acétate d'éthyle et on laisse cristalliser le produit. On obtient le produit du titre. P.f. : 111-114°C

### 68b) 3-[1-(5-éthoxycarbonylpyrid-2-yl)-4-pipéridinamino]-1-[(4-hydroxy-3-méthylsulfonyl)-phénoxy]-2-propanol

Le produit issu de l'étape précédante est traité avec CF₃COOH selon le procédé décrit dans l'exemple 26b, le produit brut étant purifié par flash-chromatographie en élunat par un mélange CH₂Cl₂/MeOH/NH₃ conc. = 90/10/1. On obtient le produit du titre.

### EXEMPLE 69

### 3-[1-(6-chloro-pyrid-2yl)-4-pipéridinylamino]-1-[-4-hydroxy-3-méthansulfonamido)-phenoxy]-2-propanol

On fait réagir le produit de l'exemple 50 avec la 4-amino-1-(6-chloropyrid-2yl)-pipéridine (préparée comme décrit dans EP 21973) selon le procédé décrit dans l'exemple 51.

On obtient le produit du titre.

### EXEMPLE 70

### 3-[1-(6-éthoxycarbonylpyrid-2yl)-4-pipéridinylamino]-1-[-4-hydroxy-3-méthansulfonamido)-phenoxy]-2-propanol

On fait réagir le produit de l'exemple 50 avec le produit de l'exemple 57 (base) dans les conditions décrites dans l'exemple 51. On obtient le produit du titre.

### EXEMPLE 71

### 3-[1-(6-chloropyrid-2-yl)-4-pipéridinylamino]-1-(4-hydroxyphénoxy)-2-propanol

En opérant comme décrit dans l'exemple 58 mais en utilisant la 4-amino-1-(6-chloropyrid-2yl)-pipéridine en lieu du produit de l'exemple 57 on obtient le produit du titre.

## Revendications

1. Composés de formule (Ia) : 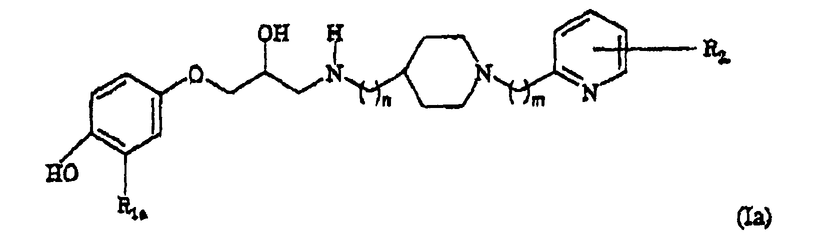 dans laquelle
R₁ₐ représente l'hydrogène, un groupe -S(O)_{z}-(C₁-C₄)Alk, un groupe -CO(C₁-C₄)Alk, un groupe -NHSO₂-(C₁-C₄)Alk, un groupe NHCO(C₁-C₄)Alk, un groupe 2-furyle ou un halogène;
R₂ représente l'hydrogène ou un groupe (C₁-C₄)Alk, un groupe (C₁-C₄)alcoxyle, un halogène, -COOH, -COO(C₁-C₄)Alk, -CN, -CONR₃R₄, -NO₂, -SO₂NH₂, -NHSO₂(C₁-C₄)Alk;
m et n sont indépendamment 0, 1 ou 2;
R₃ et R₄ représentent indépendamment l'hydrogène ou un groupe (C₁-C₄)Alk;
Z est 1 ou 2;
et leurs sels ou solvates.

2. Composé selon la revendication 1 de formule: 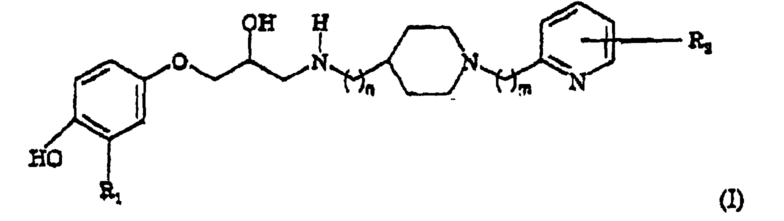 où
R₁ représente l'hydrogène, un groupe -S(O)_{z}-(C₁-C₄)Alk, un groupe -CO(C₁-C₄)Alk ou un groupe -NHSO₂-(C₁-C₄)Alk;
R₂ représente l'hydrogène ou un groupe (C₁-C₄)Alk, un groupe (C₁-C₄)alooxyle, un halogène, -COOH, -COO(C₁-C₄)Alk, -CN, -CONR₃R₄, -NO₂, -SO₂NH₂, -NHSO₂(C₁-C₄)Alk;
m et n sont indépendamment 0, 1 ou 2;
R₃ et R₄ représentent indépendamment l'hydrogène ou un groupe (C₁-C₄)Alk;
Z est 1 ou 2;
et leurs sels ou solvates.

3. Composés selon la revendication 1 ou 2 où R₂ est dans la position 5 ou 6 de la pyridine.

4. Composés selon la revendication 1 ou 2 où n et m sont zéro.

5. Composés selon la revendication 1 ou 2 où le groupe (C₁-C₄)Alk est un groupe méthyle ou éthyle.

6. Composé selon la revendication 1 ou 2 où R₂ est choisi parml -COOH, -COO(C₁-C₄)Alk, -CN, -NO₂, -CONR₂R₃ et -NHSO₂-(C₁-C₄)Alk.

7. Composé selon la revendication 1 ou 2 où R₂ est un halogène.

8. Procédé pour la préparation des composés de formule (Ia) et (I) des revendications 1 et 2 **caractérisé en ce qu'**on fait réagir un composé de formule (II) 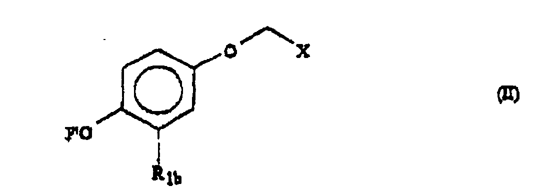 dans laquelle R₁b est R₁a ou R₁ tels qu'indiqués dans la revendication 1 ou 2, P' est un groupe protecteur et X est un groupe de formule (a) ou (b), 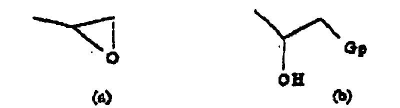 où Gp est un groupe partant, avec une amine de formule (III) 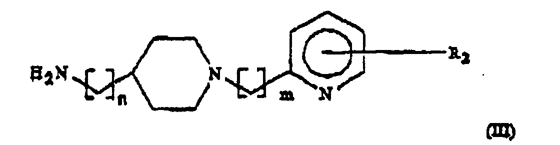 dans laquelle n, m et R₂ sont tels qu'indiqués dans la revendication 1, en clivant le groupe P' et éventuellement en transformant le composé de formule (Ia) ou (I) ainsi obtenu en un de ses sels.

9. Composition pharmaceutique contenant en tant que principe actif un composé de formula (Ia) ou (I) selon les revendications 1 à 7 ou l'un de ses sels pharmaceutiquement acceptables.

10. Utilisation d'un composé de formule (Ia) ou (I) selon les revendications 1 à 7 ou l'un de ses sels pharmaceutiquement acceptables pour la préparation de médicaments indiqués dans le syndrôme du colon irritable, ou à action modulatrice de la motricité intestinale, lipolytique, anti-obésité, anti-diabétique, psychotrope, anti-glaucomateuse, cicatrisante, anti-dépressante, comme inhibiteur des contractions utérines, comme tocolytiques pour prévenir ou retarder les accouchements précoces, pour le traitement et/ou la prophylaxie de la dysménorrhée.

## Patentansprüche

1. Verbindungen der Formel (Ia): 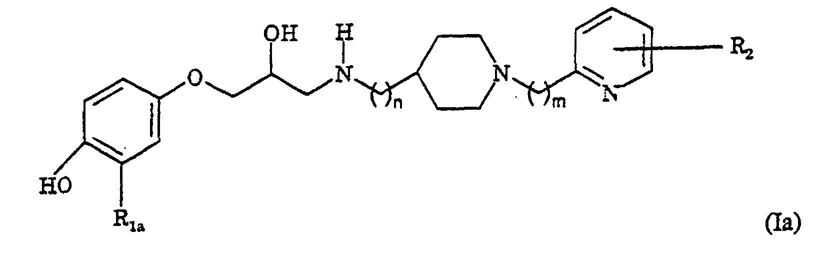 in der
R₁ₐ Wasserstoff, eine Gruppe -S(O_{)z}-(C₁-C₄)-Alk, eine Gruppe -CO(C₁-C₄)-Alk, eine Gruppe -NHSO₂-(C₁-C₄)-Alk, eine Gruppe NHCO(C₁-C₄)-Alk, eine 2-Furyl-Gruppe oder ein Halogen bedeutet;
R₂ Wasserstoff oder eine Gruppe (C₁-C₄)-Alk, eine Gruppe (C₁-C₄)-Alkoxyl, ein Halogen, -COOH, -COO(C₁-C₄)-Alk, -CN, -CONR₃R₄, -NO₂, -SO₂NH₂, -NHSO₂(C₁-C₄)-Alk bedeutet;
m und n unabhängig voneinander 0, 1 oder 2 bedeuten;
R₃ und R₄ unabhängig voneinander Wasserstoff oder eine Gruppe (C₁-C₄)-Alk bedeuten;
Z 1 oder 2 darstellt;
und deren Salze oder Solvate.

2. Verbindung der Formel (I): 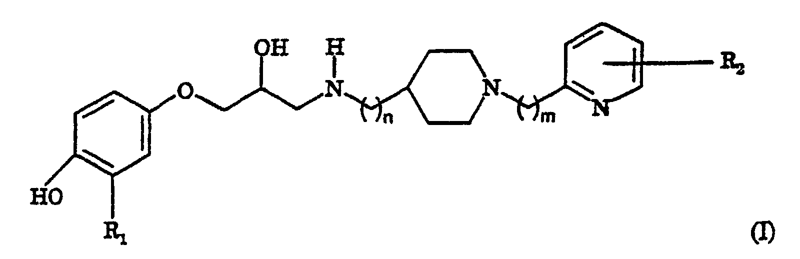 in der
R₁ Wasserstoff, eine Gruppe -S(O)_{z}-(C₁-C₄)-Alk, eine Gruppe -CO(C₁-C₄)-Alk oder Gruppe -NHSO₂-(C₁-C₄)-Alk bedeutet;
R₂ Wasserstoff oder eine Gruppe (C₁-C₄)-Alk, eine Gruppe (C₁-C₄)-Alkoxyl, Halogen, -COOH, -COO-(C₁-C₄)-Alk, CN, -CONR₃R₄, -NO₂, -SO₂NH₂, -NHSO₂(C₁-C₄)-Alk bedeutet;
m und n unabhängig voneinander 0, 1 oder 2 bedeuten;
R₃ und R₄ unabhängig voneinander Wasserstoff oder eine Gruppe (C₁-C₄)-Alk bedeuten;
Z 1 oder 2 bedeutet;
und deren Salze oder Solvate.

3. Verbindungen nach Anspruch 1 oder 2, worin R₂ in der 5- oder 6-Stellung des Pyridins steht.

4. Verbindungen nach Anspruch 1 oder 2, worin n und m null bedeuten.

5. Verbindungen nach Anspruch 1 oder 2, worin die Gruppe (C₁-C₄)-Alk eine Methyl- oder Ethyl-Gruppe ist.

6. Verbindung nach Anspruch 1 oder 2, worin R₁ ausgewählt ist aus -COOH, -COO-(C₁-C₄)-Alk, -CN, -NO₂, -CONR₂R₃ und -NHSO₂-(C₁-C₄)-Alk.

7. Verbindung nach Anspruch 1 oder 2, worin R₂ ein Halogen bedeutet.

8. Verfahren zur Herstellung der Verbindungen der Formeln (Ia) und (I) der Ansprüche 1 und 2, **dadurch gekennzeichnet, daß** man eine Verbindung der Formel (II) 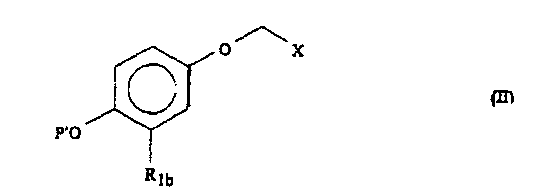 in der R₁b R₁a oder R₁ bedeutet, wie sie in den Ansprüchen 1 oder 2 definiert sind, P' eine Schutzgruppe darstellt und X eine Gruppe der Formel (a) oder (b) bedeutet 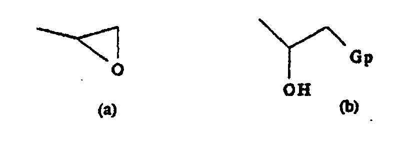 worin Gp eine austretende Gruppe darstellt, mit einem Amin der Formel (III) 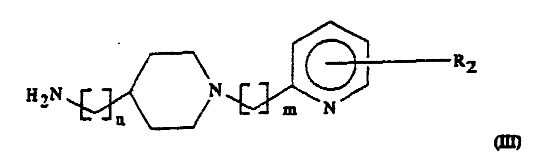 in der n, m und R₂ die in Anspruch 1 angegebenen Bedeutungen besitzen, umsetzt unter Abspalten der Gruppe P', und gegebenenfalls die in dieser Weise erhaltene Verbindung der Formel (Ia) oder (I) in eines ihrer Salze umwandelt.

9. Pharmazeutische Zubereitung enthaltend als Wirkstoff eine Verbindung der Formel (Ia) oder (I) nach den Ansprüchen 1 bis 7 oder eines ihrer pharmazeutisch annehmbaren Salze.

10. Verwendung einer Verbindung der Formel (Ia) oder (I) nach den Ansprüchen 1 bis 7 oder eines ihrer pharmazeutisch annehmbaren Salze für die Herstellung von Arzneimitteln, die gegen reizbare Colonsyndrome indiziert sind oder mit modulierender Wirkung auf die Darmmotorik, mit lipolytischer Wirkung, Wirkung gegen Fettsucht, antidiabetischer Wirkung, psychotroper Wirkung, Anti-Glaukom-Wirkung, vernarbender Wirkung, antidepressiver Wirkung, als Inhibitor für Uteruskontraktionen, als tocolytische Mittel zur Vorbeugung oder Verzögerung von Frühgeburten, zur Behandlung und/oder zur Prophylaxe von Menstruationsstörungen.

## Claims

1. Compounds of formula (Ia) : 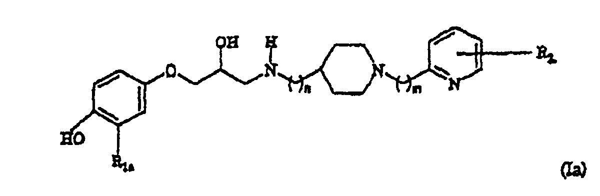 in which
R₁ₐ represents hydrogen, an -S(O)₂-(C₁-C₆)Alk group, a -CO(C₁-C₄)Alk group, an -NHSO₂- (C₁-C₄)Alk group, an NHCO(C₁-C₄)Alk group, a 2-furyl group or a halogen;
R₂ represents hydrogen or a (C₁-C₄)Alk group, a (C₁-C₄)alkoxy group, a halogen, -COOH, -COO(C₁-C₄)Alk, -CN, -CONR₃R₄, -NO₂, -SO₂NH₂ or -NHSO₂(C₁-C₄)Alk;
m and n are independently 0, 1 or 2;
R₃ and R₄ independently represent hydrogen or a (C₁-C₄)Alk group;
Z is 1 or 2;
and their salts or solvates.

2. Compounds according to Claim 1, of formula: 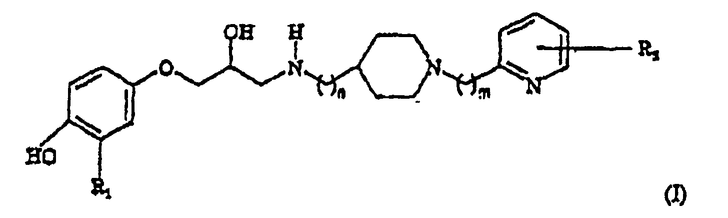 where
R₁ represents hydrogen, an -S(O)₂-(C₁-C₄)Alk group, a -CO(C₁-C₄)Alk group or an -NHSO₂- (C₁-C₄)Alk group;
R₂ represents hydrogen or a (C₁-C₄)Alk group, a (C₁-C₄)alkoxy group, a halogen, -COOH, -COO(C₁-C₄)Alk, -CN, -CONR₃R₄, -NO₂, -SO₂NH₂ or -NHSO₂(C₁-C₄)Alk;
m and n are independently 0, 1 or 2;
R₃ and R₄ independently represent hydrogen or a (C₁-C₄)Alk group;
Z is 1 or 2;
and their salts or solvates.

3. Compounds according to Claim 1 or 2, where R₂ is in the 5 or 6 position of the pyridine.

4. Compounds according to Claim 1 or 2, where n and m are zero.

5. Compounds according to Claim 1 or 2, where the (C₁-C₄)Alk group is a methyl or ethyl group.

6. Compounds according to Claim 1 or 2, where R₂ is chosen from -COOH, -COO(C₁-C₄)Alk, -CN, -NO₂, -CONR₂R₃ and -NHSO₂-(C₁-C₄)Alk.

7. Compounds according to Claim 1 or 2, where R₂ is a halogen.

8. Process for the preparation of the compounds of formulae (Ia) and (I) of Claims 1 and 2, **characterized in that** a compound of formula (II) 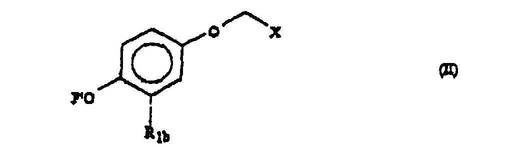 in which R_{1b} is R₁ₐ or R₁ as indicated in Claim 1 or 2, P' is a protective group and X is a group of formula (a) or (b), 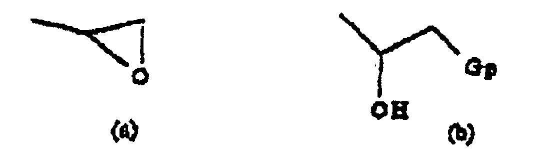 where Gp is a leaving group, is reacted with an amine of formula (III) 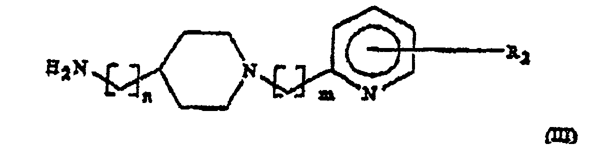 in which n, m and R₂ are as indicated in Claim 1, the group P' being cleaved and the compound of formula (Ia) or (I) thus obtained optionally being converted into one of its salts.

9. Pharmaceutical composition comprising, as active principle, a compound of formula (Ia) or (I) according to Claims 1 to 7 or one of its pharmaceutically acceptable salts.

10. Use of a compound of formula (Ia) or (I) according to Claims 1 to 7 or one of its pharmaceutically acceptable salts for the preparation of medicaments indicated in irritable bowel syndrome, or with a modulating effect on intestinal motricity or with a lipolytic, antiobesity, antidiabetic, psychotropic, antiglaucomatous, cicatrizant or antidepressant effect, as inhibitor of uterine contractions, as tocolytics for preventing or delaying premature labour, or for the treatment and/or prophylaxis of dysmenorrhoea.
